(19) Europäisches Patentamt
European Patent Office
Office européen des brevets

(11) Numéro de publication: **0 110 761**
**B1**

# (12) FASCICULE DE BREVET EUROPEEN

(45) Date de publication du fascicule du brevet: **05.02.86**

(51) Int. Cl.⁴: **C 07 C 51/60**, C 07 C 53/48, C 07 C 53/50

(21) Numéro de dépôt: **83402182.6**

(22) Date de dépôt: **10.11.83**

(54) **Procédé de préparation de chlorures d'acides alpha-chlores.**

(30) Priorité: **22.11.82 FR 8219482**

(43) Date de publication de la demande: **13.06.84 Bulletin 84/24**

(45) Mention de la délivrance du brevet: **05.02.86 Bulletin 86/6**

(84) Etats contractants désignés: **AT BE CH DE FR GB IT LI LU NL SE**

(56) Documents cités:
**DE-A-1 954 795**
**FR-A-1 488 064**
**US-A-2 361 552**
**US-A-3 636 102**

(73) Titulaire: **RHONE-POULENC SPECIALITES CHIMIQUES, "Les Miroirs" 18, Avenue d'Alsace, F-92400 Courbevoie (FR)**

(72) Inventeur: **Correia, Yves, Les Lauzières, F-04160 Château-Arnoux (FR)**
Inventeur: **Drivon, Gilles, 35, rue Alfred Guyot, F-04600 Saint-Auban (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

EP 0 110 761 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention a pour objet un procédé de préparation de chlorures d'acides α-chlorés. Elle concerne plus particulièrement la préparation de chlorures d'acides α-chlorés à partir des anhydrides des acides α-chlorés correspondants.

On connait dans l'art antérieur de tels procédés. On connait en particulier la demande de brevet allemand 2.950.155 qui décrit la préparation de chlorures d'acide par réactfon d'anhydrides d'acide sur le phosgène. Le procédé applicable à la préparation de chlorures d'acides α-chlorés peut se schématiser de la façon suivante :

$(RCHClCO)_20 + COCl_2 \rightarrow 2RCHClCOCl + CO_2$

Les inconvénients de ce procédé applicable à la préparation de chlorures d'acides α-chlorés résultent de l'utilisation du phosgène, dont le danger de mise en oeuvre est bien connu et dont le coût est élevé.

De plus, la réaction est relativement lente et demande une catalyse par divers composés aminés. Il faut encore noter la quantité importante de $CO_2$ qui se dégage (1 mole pour 2 moles de chlorure formé).

La même réaction est connue (voir par exemple le brevet des Etats-Unis d'Amerique 1.819.613) avec $SOCl_2$ au lieu de $COCl_2$. La réaction s'écrit, dans le cas de la préparation des chlorures d'acides α-chlorés:

$(RHClC\text{-}CO)_20 + SOCl_2 \rightarrow 2RHClC\text{-}COCl + SO_2$

Les inconvénients sont les mêmes que ceux soulignés ci-dessus.

On connait aussi la demande de brevet allemand 3.016.119, qui décrit la préparation de chlorures d'acides α-chlorés par réaction sur l'anhydride d'acide α-chloré correspondant, d'acide chlorhydrique.

La réaction s'écrit:

$(RCHClCO)_20 + HCl \rightarrow RCHClCOCl + RCHClCOOH$

Les grandes quantités d'acide chlorhydrique qui sont nécessaires (le procédé consiste en un stripping à l'acide chlorhydrique), posent de gros problèmes technologiques qui se répercutent sur l'économie du procédé.

On connait encore dans l'art antérieur des procédés permettant d'obtenir des chlorures d'acides α-chlorés à partir de l'acide correspondant. C'est ainsi que le brevet des Etats-Unis d'Amérique 1.805.162 décrit, notamment, la réaction de l'acide monochloracétique avec du soufre (ou du chlorure de soufre) et du chlore en présence d'un catalyseur comme $SbCl_5$, $SnCl_4$, $FeCl_3$ ou $AlCl_3$. Il se forme à côté du produit recherché du $SO_2$ et de l'HCl, selon la réaction:

$_2ClCH_2COOH + S + {}_2Cl_2 \rightarrow {}_2ClCH_2COCl + SO_2 + {}_2HCl$

ce qui pose des problèmes de pollution non négligeables.

La demande de brevet français 73 20363 publiée sous le numéro 2.232.532 décrit la preparation de chlorure d'acide monochloracetique par réaction d'acide monochloracétique et de phosgène gazeux en présence de charbon actif, le rapport molaire phosgène/acide étant supérieur à 1,5. La réaction qui peut s'écrire

$ClCH_2COOH + COCl_2 \rightarrow ClCH_2COCl + CO_2 + HCl$

montre qu'ici également se posent des problèmes de pollution importants car pour une mole de chlorure d'acide ronochloracétique, on obtient une mole de $CO_2$ et une mole d'HCl.

La demande de brevet allemand 2.943.433 décrit la rême réaction en présence de chlorure de thionyle:

$ClCH_2COOH + SOCl_2 \rightarrow ClCH_2COCl + SO_2 + HCl$

Les inconvénients sont les mêmes que dans le cas du procédé décrit dans le brevet français précite.

La demanderesse a maintenant découvert un procédé palliant les inconvénients de l'art antérieur.

L'invention a pour objet un procédé de préparation de chlorures d'acides α-chlorés caractérisé en ce que l'on fait réagir un anhydride d'acide α-chloré de formule générale :

$(R_1R_2ClC\text{-}CO)_2O$ (I)

dans laquelle $R_1$ represente l'hydrogène, le chlore ou un radical alkyle ayant de 1 à 18 atomes de carbone et dans laquelle $R_2$ représente l'hydrogène ou le chlore avec d'une part du soufre et/ou $SCl_2$ et/ou $S_2Cl_2$ et d'autre part du chlore gazeux en présence d'un acide de Lewis comme catalyseur.

L'équation du procédé selon l'invention peut s'écrire:

$(R_1R_2ClC - CO)_2O + S + {}_2Cl_2 \rightarrow 4R_1R_2ClCCOCl + SO_2$

Cette équation souligne le caractère surprenant de l'invention.

En effet, pour obtenir l'anhydride d'acide α-chloré il faut partir de l'acide α-chlore que l'on fait réagir avec de l'anhydride acétique. Donc, le procédé selon l'invention revient, par rapport aux procédés de l'art antérieur partant de l'acide α-chloré, à augmenter d'une étape le schéma réactionnel. Cet allongement permet paradoxalement une diminution importante de la quantité formée de produits secondaires, d'où diminution de la pollution, tout en ne penalisant pas le procédé sur le plan economique. En effet, pour 4 moles de chlorure d'acide α-chloré, on n'obtient qu'une mole de $SO_2$ ce qui représente une diminution de 50 % par rapport à l'art antérieur ; de plus, il faut souligner qu'il ne se forme pas d'acide chlorhydrique. Sur le plan économique, il faut noter que lorsque l'on prepare l'anhydride selon la réaction (dans le cas de l'anhydride monochloracétique)

$_2ClCH_2COOH + (CH_3CO)_2O \rightarrow (ClCH_2CO)_20 + {}_2CH_3COOH$

on peut récupérer l'acide acétique forme sous forme d'acide monochloracetique par action du chlore, selon la réaction bien connue :

$CH_3COOH + Cl_2 \rightarrow ClCH_2COOH + HCl$

Cet acide monochloracétique peut être utilisé pour la preparation de l'anhydride

monochloracétique.

Par rapport aux procédés de l'art antérieur, partant de l'anhydride d'acide chlore, le procédé selon l'invention permet de diminuer la quantité de $SO_2$ (ou de $CO_2$) de 50 % et permet de ne pas utiliser de l'acide chlorhydrique en grande quantité.

L'acide de Lewis utilisé comme catalyseur permet la stabilisation de $SCl_4$ qui se forme in situ et qui est l'agent de chloration actif. On peut utiliser soit un acide de Lewis fort, soit un acide de Lewis faible. Selon que cet acide sera fort ou sera faible, l'homme de l'art choisira une température respectivement faible ou forte à l'intérieur de la fourchette donnée ci-après.

De préférence, l'acide de Lewis est choisi parmi le groupe comprenant $FeCl_3$, $SnCl_4$, $SbCl_5$, $AlCl_3$, $AuCl_3$. Parmi ceux-ci, on préfèrera tout particulièrement mettre en oeuvre $FeCl_3$.

On utilise de préférence une quantité de catalyseur comprise entre 0,001 et 5 % molaire par rapport à l'anhydride de départ. Encore plus préférentiellement, cette quantité est comprise entre 0,05 et 2 %.

L'anhydride de départ peut contenir un certain taux d'impuretés formées lors de sa préparation ou celle de l'acide correspondant. Ces impuretés inhibent partiellement l'action complexante de l'acide de Lewis mis en oeuvre sur l'agent de chloration $SCl_4$. On obtient généralement un anhydride à 95-98 % (2 à 5 % restant étant de l'acide correspondant). Lorsque le taux d'impureté augmentera on augmentera la quantité de catalyseur (acide de Lewis) mis en oeuvre.

On utilise de préférence une quantité de soufre ou de ses dérivés chlorés telle que en fin de réaction on ait un excès d'anhydride n'ayant pas réagi, ceci pour obtenir un produit ayant une coloration satisfaisante. Cela conduit à mettre en oeuvre en début de réaction un excès ou un défaut de soufre ou de ses dérivés chlorés ($SC1_2$ et $8_2Cl_2$) compte tenu du fait que de par leur volatilité, $SCl_2$ et $S_2Cl_2$ peuvent être entrainés par le $SO_2$ formé. Cet excès ou ce défaut de S, $SCl_2$ et $S_2Cl_2$ est de préférence inférieur à 10 % en mole par rapport à la stoechiométrie. 11 est plus préférentiellement inférieur à 2 %.

On utilise de préférence une quantité de chlore gazeux telle que la quantité totale de chlore introduite ($Cl_2$, $8C1_2$) soit comprise entre 1 et 1,1 fois la stoechiométrie. Encore plus préférentiellement, elle est comprise entre 1 et 1,02 fois la stoechiometrie.

La réaction a lieu de préférence sans solvant bien que l'on puisse utiliser un solvant inerte tel que $CCl_4$.

La température de réaction est comprise de préférence entre 20 et 200°C. Pour les composés monochlorés on préfèrera opérer entre 30 et 80°C.

On opère de préférence à la pression atmosphérique bien que des pressions supérieures ou inférieures à la pression atmosphérique ne soient pas exclues du domaine de l'invention.

Le procédé de l'invention va être maintenant plus complètement décrit à l'aide des exemples qui vont suivre:

**EXEMPLE 1**

Dans un ballon équipé d'une introduction de chlore, d'une agitation, d'un condenseur refroidi à 0°C et d'une prise de température , on placé 342 g (2 moles) d'anhydride α-chloroacétique, 35 g (1,1 atome) de soufre en fleur et 1,5 g de $FeCl_3$ anhydre. On maintient la température à 70°C et on introduit en continu en 2 heures, 2,3 moles de chlore. On constate que la réaction est terminée par la coloration intense dûe au chlore dans le condenseur d'évent. On procède ensuite à la distillation du produit en remplaçant le condenseur à reflux par une colonne à distiller.

On obtient ainsi:

- 787 g (6,96 moles) de chlorure de monochloracétyle de coloration 30 Hazen et contenant les impuretés suivantes:
  . $SO_2 \leq 0,05$ % en poids
  . $SOC1_2 \leq 0,1$ % en poids
  . $SO_2Cl_2 \leq 0,3$ % en poids
  . chlorure de dichloracétyle ≤-0,4 % en poids

Le rendement molaire est de 87 %.

**EXEMPLE 2**

On procède de manière identique a l'exemple 1. On charge 2 moles (398 g) d'anhydride α-chloropropionique, 35g de soufre en fleur et 1,5 g de $FeCl_3$. On naintient une température comprise entre 60° et 70°C pendant l'introduction continue de 2,5 moles de chlore en 7 heures. On procède alors à un stripping par l'azote pendant 1 heure à température ambiante.

Le mélange réactionnel est ensuite distillé à pression atmosphérique. On obtient 475 g de chlorure d'α-chloropropionyle (108°C/721 mmHg), soit un rendement de 93,5 %.

Ce chlorure d'α-chloropropionyle a les caractéristiques suivantes:

- pureté 100 %
- coloration 5 Hazen
- teneur en soufre total ≦ 5 ppm

**EXEMPLE 3**

On charge 118 g (0,35 mole) d'anhydride α-chloro octanoïque, 6,1 g de soufre en fleur et 0,5 g de $FeCl_3$. On maintient vers 70°C pendant l'introduction lente d'environ 0,5 mole de chlore en 5 heures. Après un stripping du milieu réactionnel par l'azote, on récupère par distillation sous pression réduite, 122 g (0,62 mole), soit un rendement de 88 %, un chlorure d'α-chloro octanoyle ayant une pureté de 93 %

(chromatographie en phase vapeur) et une teneur en soufre de 300 ppm.

**Revendications**

1) Procédé de préparation de chlorures d'acides α-chlorés caractérisé en ce que l'on fait réagir un anhydride d'acide -chloré ayant pour formule:

$(R_1R_2ClC\text{-}CO)_2O$ (I)

dans laquelle $R_1$ représente l'hydrogène, le chlore ou un radical alkyle ayant de 1 à 18 atomes de carbone et dans laquelle $R_2$ représente l'hydrogène ou le chlore avec d'une part du soufre et/ou $SCl_2$ et/ou $S_2Cl_2$ et d'autre part du chlore gazeux en présence d'un acide de Lewis comme catalyseur.

2) Procédé selon la revendication précédente, caractérisé en ce que l'acide de Lewis est choisi parmi le groupe comprenant $FeCl_3$, $SnCl_4$, $SbCl_5$, $AlCl_3$ et $AuCl_3$.

3) Procedé selon la revendication 1 ou 2, caractérisé en ce que l'acide de Lewis est $FeCl_3$.

4) Procédé selon l'une quelconque des revendications precédentes, caractérisé en ce que l'acide de Lewis est utilisé en quantite comprise entre 0,001 % et 5 % molaire par rapport à l'anhydride de départ.

5) Procédé selon l'une quelconque des revendications precédentes, caractérisé en ce que l'on utilise une quantité de S et/ou $SCl_2$ et/ou $S_2Cl_2$ en defaut ou en excès par rapport à la stoechiométrie inférieure à 10 % en mole.

6) Procédé selon la revendication 5, caractérisé en ce que le défaut est compris entre 0,1 et 1 %.

7) Procédé selon l'une quelconque des revendications precédentes, caractérisé en ce que l'on met en oeuvre une quantité de chlore gazeux telle que la quantité totale de chlore introduite soit comprise entre 1 et 1,1 fois la stoechicmétrie.

8) Procédé selon l'une quelconque des revendications precédentes, caractérisé en ce que la terpérature est comprise entre 20 et 200°C.

**Patentansprüche**

1. Verfahren zur Herstellung von Chloriden α-chlorierter Säuren, dadurch gekennzeichnet, daß man ein ein α-chloriertes Säureanhydrid der Formel

$(R_1R_2ClC\text{-}CO)_20$ (I)

in der $R_1$ Wasserstoff, Chlor oder ein Alkylradikal mit 1 bis 18 Kohlenstoffatomen, und $R_2$ Wasserstoff oder Chlor bedeuten,

einerseits mit Schwefel und/oder $SCl_2$ und/oder $S_2Cl_2$ und andererseits mit gasförmigem Chlor in Gegenwart einer Lewis-Säure als Katalysator umsetzt.

2. Verfahren nach Anspruch 1, dadurch gekennzeichnet, daß die Lewis-Säure aus der Gruppe von $FeCl_3$, $SnCl_4$, $SbCl_5$, $AlCl_3$ und $AuCl_3$ ausgewählt wird.

3. Verfahren nach Anspruch 1 oder 2, dadurch gekennzeichnet, daß die Lewis-Säure $FeCl_3$ ist.

4. Verfahren nach einem der vorangehenden Ansprüche, dadurch gekennzeichnet, daß die Lewis-Säure in einer Menge zwischen 0,001 % und 5 Mol-% eingesetzt wird, bezogen auf die Ausgangsmenge an Anhydrid.

5. Verfahren nach einem der vorangehenden Ansprüche, dadurch gekennzeichnet, daß man eine Menge an S und/oder $SCl_2$ und/oder $S_2Cl_2$ im Unter- oder Überschuß, bezogen auf die stöchiometrische Menge, von weniger als 10 Mol-% einsetzt.

6. Verfahren nach Anspruch 5, dadurch gekennzeichnet, daß der Unterschuß zwischen 0,1 und 1 % beträgt.

7. Verfahren nach einem der vorangehenden Ansprüche, dadurch gekennzeichnet, daß man eine derartige Menge an gasförmigem Chlor einsetzt, daß die Gesamtmenge an eingeführtem Chlor zwischen dem 1- und 1,1-fachen der stöchiometrischen Menge liegt.

8. Verfahren nach einem der vorangehenden Ansprüche, dadurch gekennzeichnet, daß die Temperatur zwischen 20 und 200°C liegt.

**Claims**

1. A process for the preparation of α-chloroacyl chlorides, characterised in that an α-chloroacidanhydride having the formula:

$(R_1R_2ClC\text{-}CO)_2O$ (I)

in which $R_1$ denotes hydrogen, chlorine or an alkyl radical containing from 1 to 18 carbon atoms and in which $R_2$ denotes hydrogen or chlorine is reacted with, on the one hand, sulphur and/or $SCl_2$ and/or $S_2Cl_2$ and, on the other hand, gaseous chlorine in the presence of a Lewis acid as a catalyst.

2. A process according to the preceding claim, characterised in that the Lewis acid is chosen from the group comprising $FeCl_3$, SnCl4, $SbCl_5$, $AlCl_3$ and $AuCl_3$.

3. A process according to Claim 1 or 2, characterised in that the Lewis acid is $FeCl_3$.

4. A process according to any one of the preceding claims, characterised in that the Lewis acid is used in a quantity of between 0.001 mo1e% and 5 mo1e% relative to the starting anhydride.

5. A process according to any one of the preceding claims, characterised in that the quantity of S and/or $SCl_2$ and/or $S_2Cl_2$ which is used in deficiency or in excess relative to the stoichiometry is lower than 10 mole%.

6. A process according to Claim 5, characterised in that the deficiency is between 0.1 and 1%.

7. A process according to any one of the preceding claims, characterised in that a quantity of gaseous chlorine is used such that the total

quantity of chlorine introduced is between 1 and 1.1 times the stoichiometry.

8. A process according to any one of the preceding claims, characterised in that the temperature is between 20 and 200° C.